**Europäisches Patentamt**

**European Patent Office**

**Office européen des brevets**

(11) Publication number: **0 025 719**
**A2**

# EUROPEAN PATENT APPLICATION

(21) Application number: **80303246.5**

(22) Date of filing: **15.09.80**

(51) Int. Cl.³: **A 61 K 39/395, C 07 G 7/00,**
**A 61 K 35/14**

(30) Priority: **17.09.79 JP 120357/79**
**30.07.80 JP 105618/80**

(43) Date of publication of application: **25.03.81**
**Bulletin 81/12**

(84) Designated Contracting States: **BE CH DE FR GB IT LI**
**SE**

(71) Applicant: **MORISHITA PHARMACEUTICAL CO. LTD.,**
**29 Doshomachi 4-chome Higashi-ku, Osaka (JP)**

(72) Inventor: **Takagi, Takashi, 1460-122 Minamizakura**
**Yasu-cho, Yasu-gun Shiga-ken (JP)**
Inventor: **Matsuo, Toshiyasu, 615-6 Nagahara**
**Yashu-cho, Yasu-gun Shiga-ken (JP)**

(74) Representative: **Myerscough, Philip Boyd et al, J.A.Kemp**
**& Co. 14, South Square Gray's Inn, London, WC1R 5EU**
**(GB)**

(54) Gamma-globulin preparation for intravenous administration, process for production thereof and process for preparation of gamma-globulin of low anticomplementary activity.

(57) Conventional techniques of purifying gamma-globulin from human blood suffer from a variety of disadvantages, not least being problems associated with the removal of impurities of high anticomplementary activity. A gamma-globulin of an anticomplementary activity of lower than 20% ($C'H_{50}$ value) in now prepared by forming a suspension of a pH of 7.0 to 9.0 of Cohn's Fraction II for said gamma-globulin in an aqueous solution of a monosaccharide, disaccharide or sugar alcohol, adding thereto dextran of an average molecular weight of 10,000 to 70,000 to produce an aqueous 2 to 10% wt./vol. solution of dextran and, after removing the thus formed precipitate, adding ammonium sulfate to the remaining mother liquor thereby to precipitate said gamma-globulin. A gamma-globulin preparation containing a gamma-globulin of such low anticomplementary activity is stabilized by specific amounts of L-arginine, L-lysine or a pharmaceutically acceptable salt thereof. The gamma-globulin and the preparation, which may be lyophilized, can be employed for intravenous administration to humans.

- 1 -

DESCRIPTION

TITLE: GAMMA-GLOBULIN PREPARATION FOR INTRAVENOUS
ADMINISTRATION, PROCESS FOR PRODUCTION THEREOF
AND PROCESS FOR PREPARATION OF GAMMA-GLOBULIN
OF LOW ANTICOMPLEMENTARY ACTIVITY

The present invention relates to a gamma-globulin preparation suitable for intravenous administration, to a process of producing a gamma-globulin preparation suitable for intravenous administration and to a process for producing a gamma-globulin suitable for intravenous administration and of an anticomplementary activity of lower than 20% ($C'H_{50}$ value).

The method of Cohn et al. (J. Am. Chem. Soc., 68, 459 - 475 (1946) and ibid., 72, 465 - 474 (1950)) has been habitually utilized for separating and collecting gamma-globuline from human blood. However, since the gamma-globulin obtained by this method contains impurities which show a high anticomplementary activity, its use has been confined only to intramuscular injection. Even in the case of intramuscular injection, this gamma-globulin gives a topical pain. Moreover, since the gamma-globulin remains at the site of injection for a long time, satisfactorily high levels of gamma-globulin in the patient's blood are not attainable due to degradation of the gamma-globulin during its long retention at the site of injection.

In order to overcome such a defect, methods for obtaining gamma-globulin for safe intravenous administration, for instance by treating gamma-globulin with an enzyme or an acid, or by chemically modifying the gamma-globulin, have been devised. However, owing

- 2 -

to several defects caused by these treatments, such as the reduction of the half-life of the gamma-globulin and antigenicity problems, the production of a gamma-globulin with its original and native form as derived from human blood has attracted much attention. Various methods of doing this have been proposed, including using a copolymer of ethylene oxide and propylene glycol (Japanese Patent Application Laying Open No. 81519/74), using hydroxyethylstarch and polyethylene glycol (Japanese Patent Publication No. 12001/80), and using polyethylene glycol (U. S. Patent No. 4,165,370 and U. S. Patent No. 4,124,576).

It is very difficult to remove from the purified gamma-globulin the high polymeric substances which have been used in such methods to remove the impurities from the gamma-globulin whereas it is preferable for such substances to be removable completely from the purified gamma-globulin or administrable intravenously. In general, the polymeric substances used in conventional methods do not necessarily satisfy these criteria. Further, the purified gamma-globulin, when formulated as a pharmaceutical preparation by the usual methods, shows raised anticomplementary activity again. It is therefore necessary to devise a stabilizing method which inhibits the rise of the anticomplementary activity. Stabilizing techniques include the addition to the purified gamma-globulin of an amino acid, a sugar, a neutral salt or a high polymeric non-ionic surfactant of molecular weight of 2,000 to 20,000 at a relatively high concentration (Japanese Patent Applications Laying Open Nos. 47515/78 and 20124/79) and of a neutral salt, such as sodium chloride, and human serum albumin (Japanese Patent Application Laying Open No. 23115/79). In the former case, owing to the high concentration of the stabilizing agent, the formulated preparations are hypertonic and, in addition, among the additives are substances not preferable for living bodies. In the latter case, even if there is almost no fear of the thus obtained purified gamma-globulin causing transmission of serum hepatitis, however, human serum albumin is highly dangerous in causing such a disease. The use of such a dangerous stabilizing agent should be avoided. The stability of the human serum albumin also becomes a problem.

It has now been found that a gamma-globulin of very low anticomplementary activity and which fully satisfies the standard (the national standard of biological pharmaceutical preparations established by the Ministry of Welfare, the Japanese Government, which requires an anticomplementary activity of less than 20% ($C'H_{50}$ value) at a concentration of the preparation of 50 mg/ml) can be obtained by the combined use of dextran, which has been hitherto utilized in injections for humans, and of a monosaccharide, disaccharide or sugar alcohol.

The yield of the purified gamma-globulin depends on both of the kind of monosaccharide, disaccharide or sugar alcohol used, and of the conditions of fractionation. However, the yield is generally in the range of 74 to 93% of the analytically available amount of gamma-globulin.

Accordingly, in a first aspect the present invention provides a process for producing a gamma-globulin suitable for intravenous administration and of an anticomplementary activity of lower than 20% ($C'H_{50}$ value), which process comprises forming a suspension of a pH of 7.0 to 9.0 of Cohn's Fraction II for said gamma-globulin in an aqueous solution of a monosaccharide, disaccharide or sugar alcohol, adding thereto dextran of an average molecular weight of 10,000 to 70,000 to produce an aqueous 2 to 10% wt./vol. solution of dextran and, after removing the thus formed precipitate, adding ammonium sulfate to the remaining mother liquor thereby to precipitate said gamma-globulin.

Furthermore, as a result of continued studies to obtain a stabilizing agent which is harmless to living bodies and which effectively inhibits the rise of the anticomplementary activity of a pharmaceutical preparation after the production of the preparation based on purified gamma-globulin at a concentration at which the solution shows an osmotic pressure close to the physiological osmotic pressure, it has been found that L-arginine, L-lysine or a salt thereof is effective at a concentration of 0.15 to 0.3 mol/litre in stabilizing a gamma-globulin.

In a second aspect, therefore, the present invention provides a gamma-globulin preparation suitable for intravenous administration, which preparation comprises a gamma-globulin of an anticomplementary activity of lower than 20% ($C'H_{50}$ value) and from 3 to 6 mol per kg of said gamma-globulin of at least one stabilizer selected from L-arginine, L-lysine and pharmaceutically acceptable salts thereof.

Such a preparation is prepared according to the present invention by forming a solution of a pH of 6.4 to 7.2 of at least one stabilizer selected from L-arginine, L-lysine and pharmaceutically acceptable salts thereof in an aqueous physiological saline solution containing a gamma-globulin of an anticomplementary activity of lower than 20% ($C'H_{50}$ value), said stabiliser being present in an amount of from 3 to 6 mol per kg of gamma-globulin contained in said saline
solution. In a further process according to the
invention the solution that is formed contains 1.5 x
$10^2$ to 3 x $10^2$ mol of said stabilizer/$m^3$ per $10^{-3}$ kg
of gamma-globulin contained in said saline solution.
The resulting solution of either process of pH of 6.4
to 7.2 can be lyophilized. The gamma-globulin of an
anticomplementary activity of lower than 20% ($C'H_{50}$
value) that is employed in producing the preparation
can be obtained in accordance with the first aspect
of the invention.

In order to prepared the gamma-globulin of
low anticomplementary activity in accordance with the
first aspect of the invention, a raw material for
gamma-globulin, represented by Cohn's Fraction II
(loc. cit.), can be suspended in an aqueous solution
of preferably, from 3 to 15% by weight of a monosaccharide such as glucose, fructose, galactose, etc.,
a disaccharide such as lactose, maltose, etc, or a
sugar alcohol such as mannitol, xylitol, etc. at a
concentration of 2.5 x $10^{-2}$ to 6.0 x $10^{-2} m^3$/kg ( 25 to
60 ml/g), preferably 4.5 x $10^{-2}$ to 5.5 x $10^{-2} m^3$/kg
(45 to 55 ml/g) of the protein contained in the fraction.
The pH value of the aqueous suspension is adjusted to
7.0 to 9.0.

The thus prepared aqueous suspension is
subjected to fractional precipitation by adding dextran
thereto at a concentration of 2 to 10% wt./vol. Portions
of the dextran may be added in three stages, that is,
first to a concentration of 2% wt./vol., then a
concentration of 3 to 5% wt./vol. and finally to a
concentration of 6 to 10% wt./vol.. The impurities showing anticomplementary activity are removed during the course of the fractional precipitation as precipitates. The procedure up to this point may be practised at room temperature. As the final procedure, ammonium sulfate is added to the mother liquid at a concentration of 15 to 35% wt./vol. at a temperature of 0 to 10°C, preferably 0 to 5°C, to precipitate the thus purified gamma-globulin. This gamma-globulin is suitable for intravenous administration.

Although a purified gamma-globulin can be obtained at nearly the same yield by using solely dextran without any monosaccharide, disaccharide or sugar alcohol its anticomplementary activity is 25 to 30 ($C'H_{50}$) at a gamma-globulin concentration of 50 kg/m$^3$ (50 mg/ml). In other words, a product satisfying the standard for biological preparations for pharmaceutical use of anticomplementary activity of less than 20 ($C'H_{50}$) cannot be obtained. A satisfactory product requires the further presence of a monosaccharide, disaccharide or sugar alcohol. This results in a noticeable reduction of anticomplementary activity. Nevertheless, where a monosaccharide, disaccharide or sugar alcohol is used alone, no reduction of anticomplementary activity is observed. When hydroxyethyl starch is used instead of dextran, the aqueous solution becomes turbid and it is not able to purify a gamma-globulin.

The pH of the solution in the fractional precipitation step is preferably from 7.0 to 9.0. Where the pH is lower than 7.0 or higher than 9.0, the removal of impurities is insufficient and the anticomplementary activity is not sufficiently reduced. The dextran may be, for example, Dextran 10 (with an average molecular weight of 10,000), Dextran 40 (with

0025719

- 7 -

an average molecular weight of 40,000) or Dextran 70 (with an average molecular weight of 70,000). Dextran 40 is preferably utilized at a concentration of from 2 to 10% wt./vol. as regards the relationship between the anticomplementary activity and the yield of the product.

A pharmaceutical preparation containing the purified gamma-globulin can be obtained in accordance with the invention by dissolving the gamma-globulin in an aqueous physiological saline solution at a concentration of 5 to 15% wt./vol., and adding L-arginine, L-lysine or one of their salts to the solution at a concentration of $1.5 \times 10^2$ to $3.0 \times 10^2$ mol/m$^3$ (0.15 to 0.3 mol/litre) and then adjusting the pH of the mixture to 6.4 to 7.2. The preparation can be sterilized by filtering and then freeze-dried to obtain the product. The kinds, the concentration in use, and the effectiveness of the stabilizing agents are shown in Examples 1 to 3.

In addition, it has been found that the process of the second aspect of the present invention for stabilizing a pharmaceutical preparation of gamma-globulin of sufficiently low anticomplementary activity can be applied not only to a gamma-globulin prepared in accordance with the first aspect of the invention but also to a gamma-globulin prepared by the other techniques. Pharmaceutical preparations prepared using the latter type of gamma-globulin are as stable those containing gamma-globulin purified according to the first aspect of the present invention. Example 4 shows this situation.

The following Examples illustrate the present invention.

EXAMPLE 1:

Into separate $2 \times 10^{-5} m^3$ (20 ml) portions of an aqueous physiological saline solution, $10^{-3}$ kg (one gram) of the gamma-globulin, purified by the above - mentioned method and having a low anticomplementary activity was dissolved. Glycine, L-alanine, L-arginine, L-arginine hydrochloride, L-lysine, L-lysine hydrochloride, L-ornithine hydrochloride, sodium L-aspartate, sodium L-glutamate and epsilonaminocaproic acid were respectively dissolved in each solution at a concentration of $2.0 \times 10^2$ mol/m$^3$ (0.2 mol/litre).

These mixtures were respectively adjusted by an aqueous 1N solution of sodium hydroxide or hydrogen chloride to a pH of 7.0 and then were freeze dried. After one hour from the completion of freeze-drying, distilled water was added to each of the freeze-dried products. The resulting concentration of gamma-globulin was 5 to 5.2% wt./vol.. Anticomplementary activity was measured according to the method of Kabat and Mayer, described in Experimental Immunochemistry, page 225, XII Ed. 1961, after having subjected the aqueous mixture to dialysis against aqueous physiological saline solution to remove the amino acid contained therein from the consideration of the effect of the amino acid on the determination of the anticomplementary activity.

The results are shown in Table 1.

Table 1

| Additive: $2.0 \times 10^2 \text{mol}/\text{m}^3$ (0.2 mol/litre) | Anticomplementary activity $(C'H_{50})$* | |
|---|---|---|
| | Before freeze-drying | After freeze-drying |
| Not added | 12 | 47 |
| Glycine | 12 | 30 |
| L-Alanine | 12 | 44 |
| L-Arginine | 12 | 14 |
| L-Arginine hydrochloride | 12 | 14 |
| L-Lysine | 12 | 14 |
| L-Lysine hydrochloride | 12 | 14 |
| L-Ornithine hydrochloride | 12 | 32 |
| Sodium L-aspartate | 12 | 22 |
| Sodium L-glutamate | 12 | 26 |
| epsilon-aminocaproic acid | 12 | 23 |

Note:  * The figures indicate the amount of the
complement inactivated by the different
preparation of gamma-globulin expressed
in percent of the complement added in
the form of serum of a guinea-pig.

As is seen from Table 1, L-arginine, L-lysine
and their sodium salts showed a conspicuous effect in
inhibiting the rise of anticomplementary activity of
the gamma-globulin to which one of them was added as
compared with other amino acids.  In addition, L-ornithine
hydrochloride did not show any effect although L-ornithine
is an amino acid.

EXAMPLE 2:

A simlar experiment to Example 1 was carried out on the four additives which showed conspicuous effectiveness in Example 1, concerning the relationship between their effectiveness and the amount of each used. The results are shown in Table 2.

Table 2

unit: %

| Additive | | Amount added (mol/litre) $(mol/10^{-3}m^3)$ | | | | | | | |
|---|---|---|---|---|---|---|---|---|---|
| | | 0 | 0.05 | 0.10 | 0.15 | 0.20 | 0.30 | 0.40 | 0.50 |
| L-Arginine·HCl | before* | 12 | 12 | 12 | 12 | 12 | 12 | 12 | 12 |
| | after** | 50 | 39 | 36 | 18 | 14 | 13 | −*** | − |
| L-Lysine·HCl | before | 12 | 12 | 12 | 12 | 12 | 12 | 12 | 12 |
| | after | 49 | 47 | 33 | 19 | 14 | 13 | − | − |
| L-Arginine | before | 12 | 12 | 12 | 12 | 12 | 12 | 12 | 12 |
| | after | 51 | 38 | 35 | 18 | 14 | 13 | − | − |
| L-Lysine | before | 12 | 12 | 12 | 12 | 12 | 12 | 12 | 12 |
| | after | 50 | 48 | 34 | 19 | 14 | 13 | − | − |

Notes:  * : before freeze-drying,

** : after freeze-drying,

*** : The mixture could not be freeze-dried.

As is seen in Table 2, each of the amino acids and their salts was not effective in inhibiting the rise of anticomplementary activity at a concentration lower than $10^2$ mol/m$^3$ (0.1 mol/litre). At a concentration higher than $4 \times 10^2$ mol/m$^3$ (0.4 mol/litre) it became difficult to carry out freeze-drying. The optimum amount of addition of the additive was found to be in the range of $1.5 \times 10^2$ to $3.0 \times 10^2$ mol/m$^3$ (0.15 to 0.3 mol/litre).

- 11 -

0025719

EXAMPLE 3:

Four kinds of gamma-globulin preparation for pharmaceutical use were prepared and freeze-dried by the same procedures as Example 2. The concentration of amino acid or salt thereof was, however, $2.0 \times 10^2$ mol/m$^3$ (0.2 mol/litre). These preparations were kept for one month and two weeks at temperatures of 4°C and 37°C, respectively, to examine the change of their anticomplementary activity during such periods of storage. The results of the examination revealed that the anticomplementary activity did not show any change from 14 (C'H$_{50}$) even after one month at 4°C, or after two weeks at 37°C. Gamma-globulins to which one of the amino acids or salts in question have been added therefore can be stored for a long period in a stabilized state as regards anticomplementary activity.

EXAMPLE 4:

$10^{-3}$Kg (one gram) of a gamma-globulin prepared by a method other than a purification process according to the present invention, not yet stabilized and showing an anticomplementary activity of 15 (C'H$_{50}$) was dissolved in $2 \times 10^{-5}$m$^3$ (20 ml) of an aqueous physiological saline solution. L-arginine hydrochloride was added to the solution in an amount corresponding to $2.0 \times 10^2$ mol/m$^3$ (0.2 mol/litre) of solution. After adjusting the pH of the solution to 7.0 by a 1N aqueous solution of sodium hydroxide, the solution was sterilized by passing it through a millipore filter of pore size of $4.5 \times 10^{-7}$m (0.45 micron). The solution was poured into vials to be freeze-dried. The thus prepared pharmaceutical preparation showed an anticomplementary activity of 15 (C'H$_{50}$) just after the freeze-drying and also after been stored for one month at a constant temperature of 4°C.

EXAMPLE 5:

$10^{-3}$Kg (one gram) of Cohn's Fraction II was suspended in $5 \times 10^{-5}$m$^3$ (50 ml) of an aqueous 10% solution of glucose. After adjusting the pH of the suspension to 7.2 with a 1N aqueous solution of sodium hydroxide, $10^{-3}$ kg (one gram) of Dextran 40 was added. The mixture was shaken well, and left to stand for one hour. Then, it was centrifugated for 30 min at 10,000 rpm to separate the supernatant layer. $10^{-3}$ Kg (one gram) of Dextran 40 was added to the supernatant layer, and the same procedure as above was carried out. To the thus obtained supernatant layer, $10^{-3}$ kg (one gram) of Dextran 40 was added, and the same procedure was repeated once. The thus obtained supernatant layer was cooled to 4°C. $10^{-2}$ g (10 g) of ammonium sulfate was added to the cooled supernatant layer. After stirring well the mixture was left to stand for two hours and then centrifuged for 30 min at 10,000 rpm to obtain a precipitate of gamma-globulin at a yield of 76%.

After dissolving this precipitate in an aqueous physiological saline solution and subjecting the solution to dialysis against an aqueous physiological saline solution to remove ammonium sulfate, the concentration of gamma-globulin in the dialysate was adjusted to 5% by weight, and its anticomplementry activity was measured by the above-mentioned method Kabat and Mayer. The activity was 13%.

EXAMPLES 6 to 13:

The same procedures were carried out as in Example 5 except that the species of sugar and the pH of the solution of the raw material were changed. The results are shown in Table 3.

COMPARATIVE EXAMPLES 1 to 4:

In these Comparative Examples, the same procedures were carried out as in Example 5 except that Cohn's Fraction II was dissolved in water, instead

- 13 -

of in a glucose solution as in Example 5.  The
results are shown also in Table 3.

Table 3

| Example | Sugar | pH | Yield (%) | Anticomplementary activity $(C'H_{50})$ |
|---|---|---|---|---|
| 6 | glucose | 7.7 | 82 | 14 |
| 7 | glucose | 8.2 | 75 | 11 |
| 8 | lactose | 7.7 | 93 | 13 |
| 9 | fructose | 7.7 | 74 | 15 |
| 10 | galactose | 7.7 | 79 | 5 |
| 11 | maltose | 7.7 | 76 | 11 |
| 12 | mannitol | 7.7 | 79 | 7 |
| 13 | xylitol | 7.7 | 79 | 11 |
| Com. 1* | none | 7.2 | 80 | 25 |
| Com. 2 | none | 7.7 | 72 | 30 |
| Com. 3 | none | 8.2 | 63 | 28 |
| Com. 4 | none | 8.7 | 74 | 28 |

Note: "Com." means Comparative Example

EXAMPLE 14:

$1.2 \times 10^{-2}$ Kg (12 g) of powdery Cohn's Fraction
II (containing $1.03 \times 10^{-2}$ kg (10.3 g) of globulin) were
dissolved in $4 \times 10^{-4} m^3$ (400 ml) of aqueous 12% by weight
solution of mannitol.  After adjusting the pH of the
solution to 7.9, $1.46 \times 10^{-4} m^3$ (146 ml) of an aqueous
30% by weight solution of Dextran 40 were added.  The
mixture was stirred for 30 min.  After two hours of
standing still, the mixture was centrifuged for 20 min
at 7,000 rpm.  The supernatant layer was cooled to 4°C.

$1.63 \times 10^{-1}$ Kg (163 g) of ammonium sulfate was slowly added to the cooled supernatant. After leaving the mixture still over a night, it was centrifuged again for 15 min at 7,000 rpm. The precipitate obtained was suspended in $4 \times 10^{-4} m^3$ (400 ml) of an aqueous 40% by weight solution of ammonium sulfate, and after one hour of standing still, the aqueous suspension was centrifuged for 15 min at 7,000 rpm to obtain gamma-globulin, at a yield of 82%, as a precipitate.

After dissolving this precipitate in about $10^{-4} m^3$ (100 ml) of an aqueous physiological saline solution and then dialyzing the solution against a physiological saline solution for 24 hours, the gamma-globulin concentration of the dialysate was adjusted to be 5% by weight. Then, after adding $2.0 \times 10^2$ $mol/m^3$ (0.2 mol/litre) equivalent of L-arginine hydrochloride and adjusting the pH of the mixture to 7.0, the mixture was centrifuged for 20 min at 7,000 rpm. The centrifugate was sterilized by passing through a millipore filter of $4.5 \times 10^{-7} m$ (0.45 micron) in pore size, and then poured into $2 \times 10^{-5} m^3$ (20 ml) vials to be freeze-dried. The anticomplementary activity of the thus obtained product was 12 ($C'H_{50}$). According to the result of a test carried out by the method of inhibiting the aggregation of erythrocytes, the value of antibody of measles expressed by the highest dilution to cause aggregation was 1024. These two values were the same as those before freeze-drying.

EXAMPLE 15:

Another freeze-dried product according to the present invention was obtained by the same procedures as in Example 1 except that $2.0 \times 10^2$ $mol/m^3$ (0.2 mol/

litre) equivalent of L-lysine hydrochloride was employed instead of L-arginine hydrochloride as in Example 14. The anticomplementary activity activity and the value of antibody of measles of this preparation were 12 ($C'H_{50}$) and 1024 (times), respectively which were the same as those before freeze-drying.

The thus prepared pharmaceutical preparation of gamma-globulin can be safely administered intravenously as is verified by the results of the following acute toxicity test:

Acute toxicity test:

Three pharmaceutical preparations of gamma-globulin were prepared - two in accordance with Examples 14 and 15 and the third by following the procedures in Example 14 except using L-arginine instead of its hydrochloride. These preparations were dissolved in distilled water for injection at a concentration of $10^2$ kg/m$^3$ (2.0 g/ 20 ml). Each solution was injected into the groups of mice intra-caudalvenously at respective rates of $3.2 \times 10^{-5}$, $4.0 \times 10^{-5}$ and $5.0 \times 10^{-5}$ m$^3$/kg (32, 40 and 50 ml/kg). Each group of mice was composed of 5 animals. The state, behaviour and mortality, if any, of the treated mice and control were observed for 7 days. No abnormal findings or mortality occurred.

It will be appreciated that the dose of the pharmaceutical preparation of gamma-globulin according to the present invention for use in intravenous administration depends on the age, the physical condition, etc. of the subject. However, usually an amount of $1.6 \times 10^{-3}$ to $6.0 \times 10^{-3}$ kg (1.6 to 6.0 g) [$10^{-3}$ to $2.5 \times 10^{-3}$ kg (1,000 to 2,500 mg) as gamma-globulin] and $7.5 \times 10^{-5}$ to $3.6 \times 10^{-4}$ kg ( 75 to 360 mg) [$5.0 \times 10^{-5}$ to $1.5 \times 10^{-4}$ kg ( 50 to 150 mg)

- 16 -

as gamma-globulin] for an adult and a child, respectively, is used at a time.

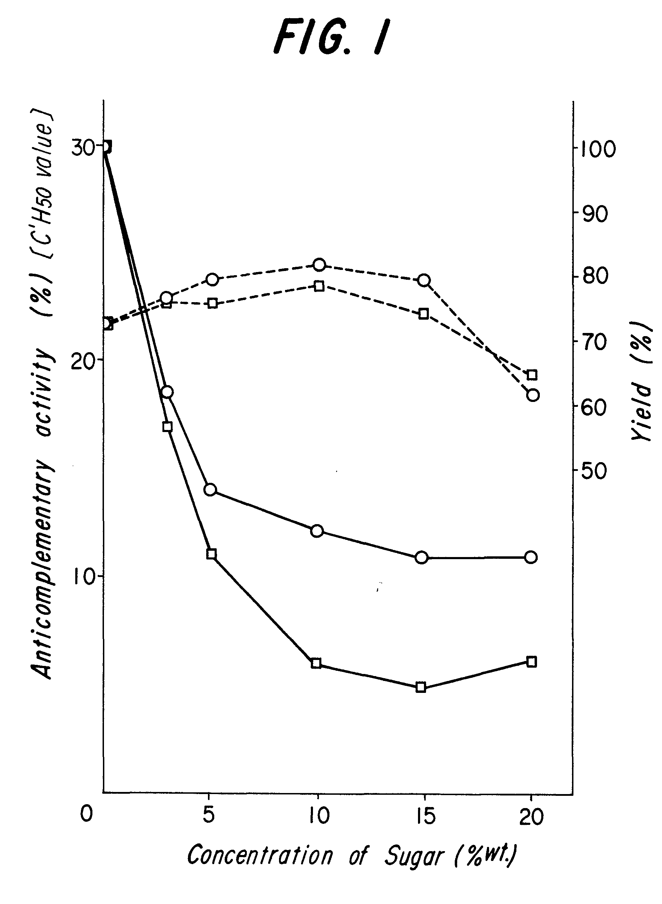

The Figure of the accompanying drawing shows that the relationships (1) between the yield of gamma-globulin and the concentration of a sugar, glucose or mannitol, and (2) between the anticomplementary activity of gamma-globulin and the concentration of the glucose or mannitol  The gamma-globulin was obtained by the procedures of Example 5 at a pH of 7.7.  The white circles indicate the level of glucose, the white squares indicate the level of mannitol, the solid line indicates the anticomplementary activity and the dotted line indicates the yield of gamma-globulin.

- 17 -

CLAIMS

1.  A gamma-globulin preparation suitable for intravenous administration, which preparation comprises a gamma-globulin of an anticomplementary activity of lower than 20% ($C'H_{50}$ value) and from 3 to 6 mol per kg of said gamma-globulin of at least one stabilizer selected from L-arginine, L-lysine and pharmaceutically acceptable salts thereof.

2.  A preparation according to claim 1 which is lyophilized.

3.  A process for preparing a gamma-globulin preparation suitable for intravenous administration, which process comprises forming a solution of a pH of 6.4 to 7.2 of at least one stabilizer selected from L-arginine, L-lysine and pharmaceutically acceptable salts thereof in an aqueous physiological saline solution containing a gamma-globulin of an anticomplementary activity of lower than 20% ($C'H_{50}$ value), said stabilizer being present in an amount of from 3 to 6 mol per kg of gamma-globulin contained in said saline solution.

4.  A process for preparing a gamma-globulin preparation suitable for intravenous administration, which process comprises forming a solution of a pH of 6.4 to 7.2 of at least one stabilizer selected from L-arginine, L-lysine and pharmaceutically acceptable salts thereof in an aqueous physiological saline solution containing a gamma-globulin of an anticomplementary activity of lower than 20% ($C'H_{50}$ value) to produce a solution containing $1.5 \times 10^{2}$ to $3 \times 10^{2}$ mol of said

stabilizer/m$^3$ per $10^{-3}$ kg of gamma-globulin contained in said saline solution.

5. A process according to claim 3 or 4 wherein said gamma-globulin has been obtained by forming a suspension of a pH of 7.0 to 9.0 of Cohn's Fraction II for said gamma-globulin in an aqueous solution of a monosaccharide, disaccharide or sugar alcohol, adding thereto dextran of an average molecular weight of 10,000 to 70,000 to produce an aqueous 2 to 10% wt./vol. solution of dextran and, after removing the thus formed precipitate, adding ammonium sulfate to the remaining mother liquor thereby to precipitate said gamma-globulin.

6. A process according to any one of claims 3 to 5 wherein the resulting solution of a pH of 6.4 to 7.2 is lyophilized.

7. A process for producing a gamma-globulin suitable for intravenous administration and of an anticomplementary activity of lower than 20% (C'H$_{50}$ value), which process comprises forming a suspension of a pH of 7.0 to 9.0 of Cohn's Fraction II for said gamma-globulin in an aqueous solution of a monosaccharide, disaccharide or sugar alcohol, adding thereto dextran of an average molecular weight of 10,000 to 70,000 to produce an aqueous 2 to 10% wt./vol. solution of dextran and, after removing the thus formed precipitate, adding ammonium sulfate to the remaining mother liquor thereby to precipitate said gamma-globulin.

# FIG. 1